Europäisches Patentamt

European Patent Office

Office européen des brevets

(19)

(11) EP 1 504 717 A1

(12) EUROPEAN PATENT APPLICATION

(43) Date of publication:
**09.02.2005 Bulletin 2005/06**

(51) Int Cl.[7]: **A61B 5/053, A61B 10/00**

(21) Application number: **04017890.7**

(22) Date of filing: **28.07.2004**

| | |
|---|---|
| (84) Designated Contracting States:<br>**AT BE BG CH CY CZ DE DK EE ES FI FR GB GR HU IE IT LI LU MC NL PL PT RO SE SI SK TR**<br>Designated Extension States:<br>**AL HR LT LV MK** | (72) Inventor: **Takehara, Tomoko, c/o Tanita Corp.** **Tokyo (JP)** |
| (30) Priority: **07.08.2003 JP 2003288467** | (74) Representative: **Grünecker, Kinkeldey,** **Stockmair & Schwanhäusser Anwaltssozietät** **Maximilianstrasse 58** **80538 München (DE)** |
| (71) Applicant: **TANITA CORPORATION**<br>**Tokyo (JP)** | |

(54) **Device for prompting to control physical condition for woman**

(57)    Disclosed is a device for prompting to control physical condition for a woman, comprising: an impedance measurement unit; a body weight input unit; a total body water calculation unit; a total body water storage unit; and a physical condition judgment unit. According to the present invention said impedance measurement unit measures bioelectrical impedance of a woman under test, said body weight input unit enters body weight of the woman under test, and said total body water calculation unit calculates total body water of the woman under test, based on the measured bioelectrical impedance and the entered body weight. Furthermore, said total body water storage unit stores the calculated total body water of the woman under test, and said physical condition judgment unit judges any change in physical condition of the woman that would be occurred at the period of a month, based on the transition in total body water stored in said storage unit.

FIG. 1

| MENSTRUATION START | MENSTRUATION END | OVULATION | | PMS | MENSTRUATION START |

MENSTRUATION TERM

GOOD-CONDITION TERM

PRESERVATION TERM

B1

BAD-CONDITION TERM

TOTAL BODY WATER

0          7          14          21          28

(NUMBER OF DAYS)

EP 1 504 717 A1

**Description**

**Background of the Invention:**

**Field of the Invention:**

The present invention relates to a device for prompting to control physical condition for a woman, which is effective to judge physical condition of a woman that would be occurred at the period of a month, such as ovulation day, PMS (Premenstrual Syndrome) term, menstruation term, child-bearing term, etc. and to judge presence of swell that tends to be occurred in the PMS term.

**Prior Art:**

As is well known, physical condition of a woman that would be occurred at the period of a month is intimately related to her basic body temperature in such manner that the basic body temperature shifts from low temperature term to high temperature term after the ovulation day, and then, gradually proceeds from low temperature term to high temperature term after the menstruation start day.

In general, the physical condition of a woman within a month has been described as divided into four terms: during-menstruation term; post-menstruation term; post-ovulation term; and pre-menstruation term. Actually, it has been found that both mind and body of a woman is delicately changed for each of those terms. In particular, during the interval from pre-menstruation to during-menstruation term, the body weight of a woman may be increased by 1 to 3 kg.

The term of one week before menstruation is called PMS (Premenstrual Syndrome) term during which many of women are suffered from any uncomfortable condition such as headache, getting nervous, stomachache, etc. In particular, there is such hypothesis that any swell tends to be occurred within the PMS term to storage body water in the body and is considered as the cause of headache and stomachache.

Weight reduction due to exercise and diet, if performed in the PMS term of a female person, may lead to accumulation of stress and lower efficiency in weight reduction. In addition, it may lead to over-eating and drinking as the counteraction, which involves any adverse effect to the person.

In order to mitigate uncomfortable condition in the PMS term it has been found necessary that the female person recognizes that she now belongs to the PMS term and positively considers that such uncomfortable condition is a kind of evidence for good health.

If change in physical condition is due to menstruation it is self-evident.

A device has been proposed in which physical condition of a woman that would be occurred at the period of a month is judged on the basis of bioelectrical impedance. More particularly, by paying attention to change in body water mass in a woman for menstruation period, bioelectrical impedance is measured to estimate body water mass of the woman and the transition in total body water is used for judgment of menstruation period (see e.g. Patent Document 1).

There is another device in which bioelectrical impedance is measured for calculating total body water. The calculated total body water is graphically displayed (see e.g. Patent Document 2).

An additional device has been available in which bioelectrical impedance is measured for calculating total body water and for judging the condition of swell (see e.g. Patent Document 3). A further device has been developed in which bioelectrical impedance is measured with input current at multiple frequencies to measure body water mass for each of intra-cellular water and extra-cellular water. Then, the ratio therebetween is used for judgment of total body water (see e.g. Patent Document 4).

Patent documents associated with the present invention are:

Patent Document 1: Japanese Patent Laid-Open No. 2001-78977
Patent Document 2: Japanese Patent Laid-Open No. 11-318845
Patent Document 3: Japanese Patent Laid-Open No. 2001-198098
Patent Document 4: Japanese Patent Laid-Open No. 2002-45346

The prior art devices for prompting to effectively control the physical condition of a woman by determining the menstruation period based on change in bioelectrical impedance can know to which term she now belongs from among several terms within a menstruation period. However, bioelectrical impedance tends to be changed depending on many factors such as change in posture, variation in body temperature, etc., and therefore, degree of precision with which the menstruation period is determined on the basis of transition in bioelectrical impedance is not so higher, which makes actual use of the device impractical.

The prior art body water meters can know total body water, intra-cellular and extra-cellular water, and a time series of change in total body water, which simply indicate the absolute amount at the time of measurement, but are not useful indices for use in control of health and physical condition. Furthermore, the ratio of intra-cellular to extra-cellular water and ratio of extra-cellular water to total body water are compared to their previous ones to judge the degree of swell. However, a person may feel or may not feel the swell, even if the numerical value indicating the swell is same. Moreover, there is difference in the degree that the person can feel the swell depending on the different physical condition of the person and the different characteristics of the persons who are likely to or not likely to feel the swell. In other words, even if it is judged to have swollen portion with the numerical value, there may be case where the person can't feel the swell, and consequently, the person has suspicious for result of judgment.

In view of the above the present invention aims at solving the prior art problems, as described above, and at providing a new and improved device for prompting a female user to know her total current physical condition by judging any change in physical condition that would be occurred at the period of a month with higher precision and by displaying information about indices that are likely to be changed in the body of the female user such as body water and swell.

**Summary of the Invention:**

According to one aspect of the present invention there is provided a device for prompting to control physical condition for a woman, comprising: an impedance measurement unit; a body weight input unit; a total body water calculation unit; a total body water storage unit; and a physical condition judgment unit, wherein

said impedance measurement unit measures bioelectrical impedance of a woman under test,

said body weight input unit enters body weight of the woman under test,

said total body water calculation unit calculates total body water of the woman under test, based on the measured bioelectrical impedance and the entered body weight,

said total body water storage unit stores the calculated total body water of the woman under test, and

said physical condition judgment unit judges any change in physical condition of the woman that would be occurred at the period of a month, based on the transition in total body water stored in said storage unit.

According to one embodiment of the present invention the device further comprises a bioelectrical impedance correction unit, wherein

said bioelectrical impedance correction unit corrects the measured bioelectrical impedance with the body weight entered by said body weight input unit, and

said total body water calculation unit calculates total body water of the woman under test, based on the corrected bioelectrical impedance and the entered body weight.

According to another embodiment of the present invention the device further comprises a bioelectrical impedance storage unit, wherein

said bioelectrical impedance storage unit stores the measured bioelectrical impedance, and

said physical condition judgment unit judges any change in physical condition of the woman that would be occurred at the period of a month, based on the transition in total body water and bioelectrical impedance stored in said storage unit.

According to further embodiment of the present invention the device further comprises a bioelectrical impedance correction unit, wherein

said bioelectrical impedance correction unit corrects the measured bioelectrical impedance with the body weight entered by said body weight input unit,

said bioelectrical impedance storage unit stores the corrected bioelectrical impedance, and

said physical condition judgment unit judges any change in physical condition of the woman that would be occurred at the period of a month, based on the transition in total body water and corrected bioelectrical impedance stored in said storage unit.

According to yet further embodiment of the present invention said body weight input unit is a body weight measurement unit so that the measured body weight is used for the entered body weight.

According to yet further embodiment of the present invention said physical condition judgment unit judges an occurrence of swell by comparison between the measured total body water and the stored total body water.

According to yet further embodiment of the present invention the device further comprises a display unit, wherein

said display unit displays the judged physical condition together with the current total body water.

According to yet further embodiment of the present invention the device further comprises an input unit, wherein

said input unit enters data of menstruation start day, and

said physical condition judgment unit judges any change in physical condition of the woman that would be occurred at the period of a month, based on the entered data of menstruation start day.

According to yet further embodiment of the present invention said physical condition judgment unit judges any change in physical condition of the woman that would be occurred at the period of a month, by comparison of the current total body water with the average of previously measured total body water.

According to yet further embodiment of the present invention said physical condition judgment unit judges any change in physical condition of the woman that would be occurred at the period of a month, by comparison of the current total body water with the average of previously measured total body water, and by comparison of current bioelectrical impedance with the average of previously measured bioelectrical impedance.

According to yet further embodiment of the present invention the average of total body water used by said physical condition judgment unit is an average of total body water after termination of the current menstruation, and if there is an increase of not less than the predefined value, as indicated by comparison of the current total body water with that average of the total body water, it is determined that ovulation has been occurred.

According to yet further embodiment of the present invention the average of total body water used by said physical condition judgment unit is an average of total body water after ovulation, and if there is an increase of not less than the predefined value, as indicated by comparison of the current total body water with that average of the total body water, it is determined that physical condition is the one that is occurred immediately before the occurrence of next menstruation.

According to yet further embodiment of the present invention said physical condition judgment unit anticipates the next ovulation day and the next menstruation start day using previous data of number of days for menstruation period.

A device for prompting to control physical condition of a woman according to the present invention is configured to judge physical condition of the woman that would be occurred at the period of a month, based on the transition in total body water. The device of the present invention is advantageous in that judgment for physical condition of the woman can be done with higher precision, because an index that is changed according to the periodical change in physical condition within a menstruation period of the woman, i.e. total body water, is directly used for judgment.

Furthermore, the device for prompting to control physical condition of a woman according to the present invention is configured to judge physical condition of the woman that would be occurred at the period of a month, based on the transition in two indices: total body water; and bioelectrical impedance. Then, judgment for physical condition of the woman can be done with more higher precision.

Moreover, the device for prompting to control physical condition of a woman according to the present invention is configured to correct bioelectrical impedance that is an index used in judgment for physical condition of the woman that would be occurred at the period of a month, depending on change in body weight. Accordingly, judgment for physical condition of the woman can be done with more higher precision.

In addition, the device for prompting to control physical condition of a woman according to the present invention is configured to judge presence of swell, based on body water mass at the time of measurement, in addition to judgment for physical condition of the woman that would be occurred at the period of a month. Then, the female user can know whether any swell is present or not before menstruation. It is effective in that the female user can know her own physical condition more sufficiently.

Furthermore, the device for prompting to control physical condition of a woman according to the present invention is configured to display measurement value of total body water, in addition to judgment for physical condition of the woman that would be occurred at the period of a month. Then, it is useful in that the female user can understand the total body water within her own body.

## Brief Description of the Drawings:

The present invention will now be described in more detail with reference to the accompanying drawings, in which:

Fig. 1 is a graph representing the transition in bioelectrical impedance "BI" and total body water within a menstruation period of a woman;
Fig. 2 is an external view illustrating a device for prompting to control physical condition for a woman according to an embodiment of the present invention;
Fig. 3 is a block diagram illustrating internal connections of the device according to the present invention, as shown in Fig. 2;
Fig. 4 is a flow chart illustrating operation of the device according to the present invention;
Fig. 5 is another flow chart illustrating operation of the device according to the present invention;
Figs. 6A to 6F are examples of information displayed on a display screen of the device according to the present invention;
Figs. 7A to 7F are other examples of information displayed on a display screen of the device according to the present invention;
Figs. 8A and 8B are further examples of information displayed on a display screen of the device according to the present invention;
Fig. 9 is an electrical equivalent circuit for cells in a human body on the basis of which the present invention operates;
Fig. 10 is a view illustrating a vector locus of bioelectrical impedance for a human body for explanation of bioelectrical impedance measurement used in the present invention; and
Fig. 11 is a graph illustrating the relation between 0 Hz frequency, "∞" frequency and characteristic frequency.

## Description of the Preferred Embodiments:

Before detailed description of a device for prompting to control physical condition of a woman according to the present invention the relationship between bioelectrical impedance ("BI") and body water mass and physical condition of a woman that would be occurred at the period of a month will be described, which the present inventor has found by repeating measurement applied to a plurality of female persons under test and on the basis of which the device of the present invention performs judgment, as described later.

The relationship between "BI" and physical condition of a woman that would be occurred at the period of a month is disclosed in said Japanese Patent Laid-Open No. 2001-78977, but it is briefly described here. In this example, the case of a woman under test whose menstruation period is 28 days is described with the following definition: menstruation term has 7 days after the menstruation start day; good-condition term has 7days before the ovulation day; preservation term has 7 days after the ovulation day; and bad-condition term has 7 days before the next menstruation start day.

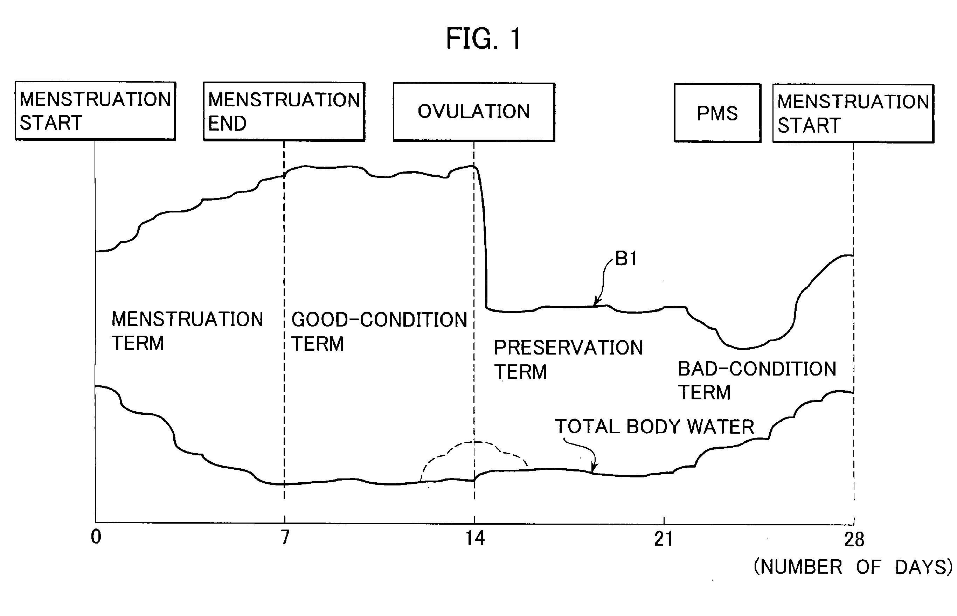

Fig. 1 is a graph representing the transition in "BI" within a menstruation period of a woman and the transition in total body water calculated using "BI" and body weight as parameters. "BI" is started to increase just before the menstruation, is kept higher during the menstruation term, and is continuously kept higher in the good-condition term after termination of the menstruation. Thereafter, "BI" is decreased after the ovulation day and is kept lower in the preservation term. Then, in the bad-condition term, "BI" tends to gradually be increased.

Then, description will be made in view of change in basic body temperature and total body water in the body of a woman under test. It has been said that water is accumulated in the body of the woman in the bad-condition term before the menstruation to create a swell. It is considered as the normal function of the body in preparation for the next menstruation. According to such phenomenon the total body water is increased from preservation term through the bad-condition term up to menstruation start day, but thereafter, it is gradually decreased. Reduction in body water mass continues in the subsequent good-condition term and generally reaches the minimum accumulation in the vicinity of the ovulation day. Increase / decrease in total body water are directly related to increase / decrease in body weight of the woman. In particular, a woman having good health tends to have the maximum body weight in the vicinity of menstruation start day, but to have the minimum body weight in the vicinity of ovulation day.

Increase in total body water means decrease in bioelectrical impedance "BI". In particular, "BI" is slightly decreased before the menstruation, but in the vicinity of the menstruation start day, it restores to substantially same value as that in the preservation term, and thereafter, it is increased with decrease in total body water. It is considered that an occurrence of swell before and during the menstruation does not apply to all the women, however many of the women tend to have swell, but with difference to some degree.

It has been said that the basic body temperature of the woman is increased after the ovulation day. Increase in basic body temperature means decrease in bioelectrical impedance "BI". Thus, "BI" becomes decreased after the ovulation day across which the good-condition term shifts to the preservation term.

Thereafter, "BI" is kept lower in the preservation term, which then proceeds to the bad-condition term.

In view of only change in total body water calculated based on "BI" and body weight, the total body water is decreased after the menstruation start day, becomes stable in the good-condition term after termination of menstruation, then slightly increased in the preservation term as compared to that in the good-condition term, and then, further increased in the bad-condition term.

It is noted that "BI" becomes changed to lower value after the ovulation day, but the body water mass has no substantial change. This is considered to relate to the fact that the body weight is at minimum upon the ovulation day. The total body water shown is calculated by "TBW" equation, as described later, but lower value of "BI" is offset due to effect of decrease in body weight. Accordingly, the calculated total body water has no significant change.

However, some female persons may have temporal slight increase in total body water before and after the ovulation, as shown by a broken line in the graph of Fig. 1 illustrating the transition in total body water. This is due to various condition associated with the ovulation and is considered to relate to body characteristic of the female person under test.

In view of the relation between "BI" and body water mass, as described above, and the general calculation of number of days for a menstruation period of a woman, judgment of physical condition of the woman that would be occurred at the period of a month is performed with the following definition:

- The menstruation term has 7days after menstruation start day and it is followed by the good-condition term;
- After termination of the menstruation term, if "BI" becomes changed from higher value to lower value or any temporal increase in body water is observed, then it is considered that the ovulation has been occurred; and
- If increase in total body water or decrease in "BI" is observed at approx. 7 days after the ovulation then it is considered that the woman has entered the bad-condition term.

By performing judgment according to the present invention in such manner, physical condition of the woman that would be occurred at the period of a month can be judged with higher precision.

Furthermore, measurement and judgment for total body water and swell are performed, and the results thereof are announced.

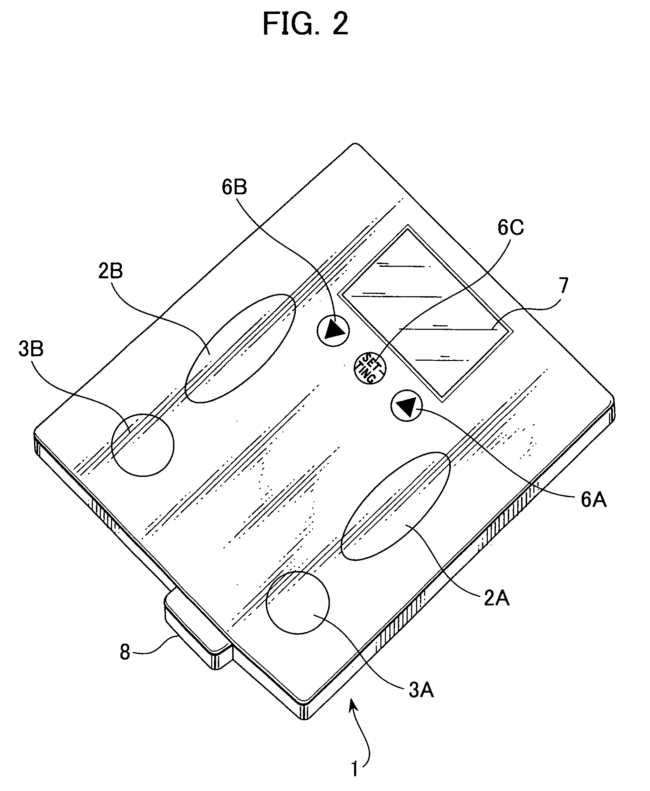

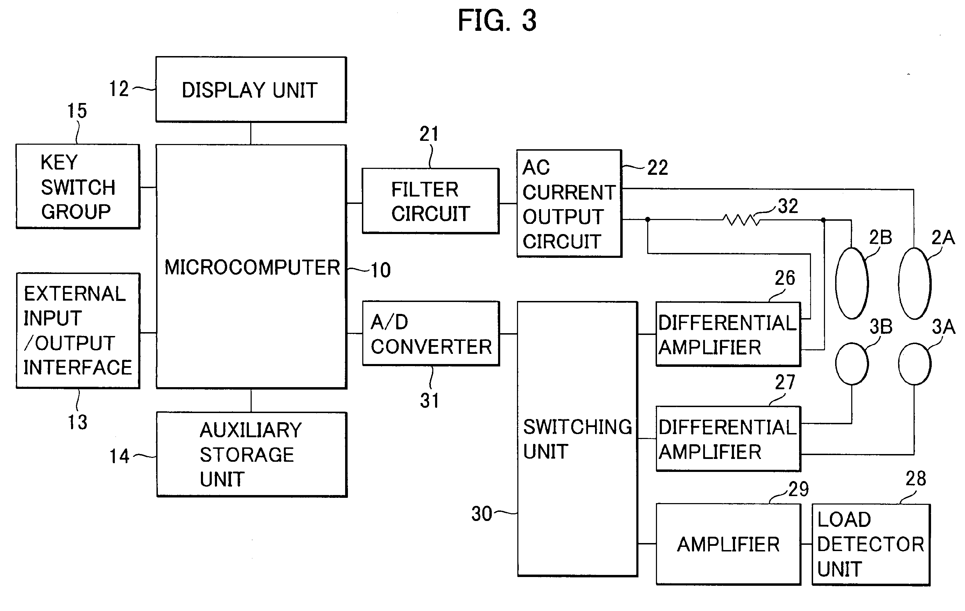

Fig. 2 is an external view illustrating a device 1 for prompting to control physical condition for a woman according to an embodiment of the present invention. Fig. 3 is a block diagram illustrating internal connections of the device 1 for prompting to control physical condition for a woman, as shown in Fig. 2.

The device 1 for prompting to control physical condition for a woman comprises a set of four electrodes: current supplying electrodes 2A, 2B and voltage measurement electrodes 3A, 3B, which are provided on a top surface of the device 1 for measuring impedance between both feet of a female user under test.

The device 1 further comprises an input unit including three switches: an up-switch 6A; a down-switch 6B; and a setting switch 6C. A display unit 7 is provided on the device 1 for displaying the entered personal body information, the measured body weight, and the calculated result.

Furthermore, a power switch 8 is provided on a front side surface of the device 1.

Referring to Fig. 3, the device 1 for prompting to control physical condition for a woman comprises a microcomputer 10 incorporating therein: a CPU for controlling measurement and judgment and for conducting arithmetic operation; a ROM for storing control and arithmetic operation program, constants, etc.; a RAM for temporally storing the calculation result, an externally retrieved program, parameters, etc.; a timer; an I/O port; and the like. The microcomputer 10 acts as both a total body water calculation unit and a physical condition judgment unit.

Furthermore, the device 1 comprises: a display unit 7 for displaying measurement condition and judgment result; an external input/output interface 13 for outputting judgment result to some external elements and for inputting control instruction and parameters from the external elements; an auxiliary storage unit 14 that is a non-volatile memory capable of storing, retrieving and updating measurement parameters, etc.; and a key switch group 15 for inputting control instructions, etc. to the device 1, which consists of: an up-switch 6A; a down-switch 6B; a setting switch 6C; and a power switch 8.

Additionally, the following elements are provided in the device 1: a filter circuit 21 for filtering a signal output from the microcomputer 10 to produce a living body applied signal; an AC current output circuit 22 for converting the signal output from the filter 21 into a fixed RMS signal; a reference resistor 23 connected to one output terminal of the AC current output circuit 22 for detecting a current through a person under test; a current supplying electrode 2B connected via the reference resistor 23 to one output terminal of the AC current output circuit 22 for supplying a measurement current to the person under test; a current supplying electrode 2A connected to another output terminal of the AC current output circuit 22 for supplying the measurement current to the person under test; a differential amplifier 26 for detecting any voltage difference across the reference resistor 23; voltage measurement electrodes 3A, 3B for detecting an electric potential between two points on the person under test; a differential amplifier 27 connected to the voltage measurement electrodes 3A, 3B for detecting an electric potential difference therebetween; a load detector unit 28 for measuring body weight of the person under test; an amplifier 29 for amplifying the signal output from the load detector unit 28; a switching unit 30 for selecting any one of the output signals from the differential amplifiers 26, 27 and the amplifier 29 under the control of the microcomputer 10; and an A/D converter 31 for converting an analog output signal from the switching unit 30 into a digital signal and for sending it to the microcomputer 10.

Now, operation of the device 1 for prompting to control physical condition for a woman according to the present invention will be described.

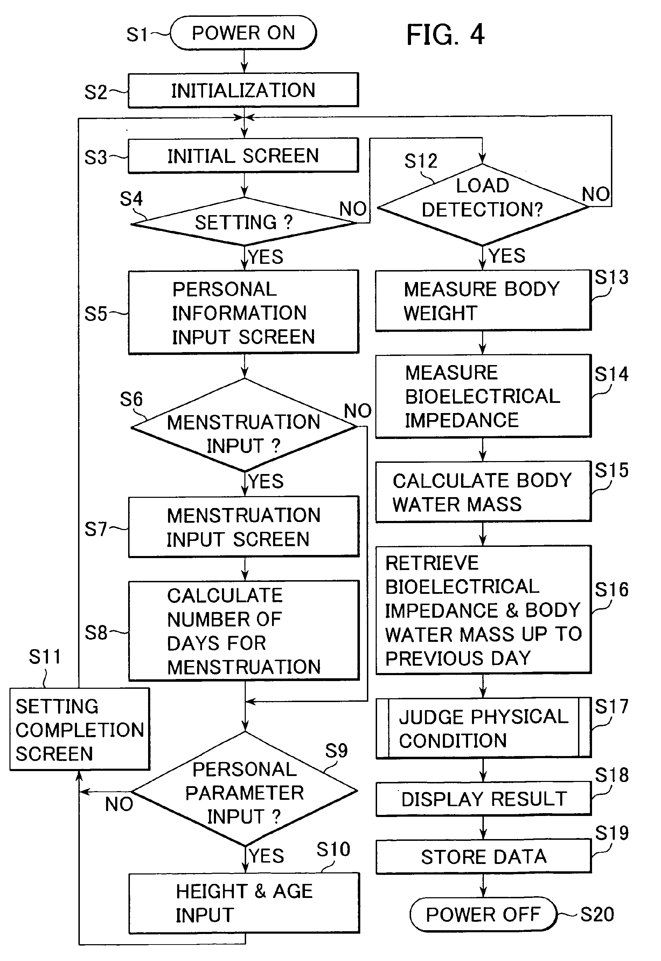

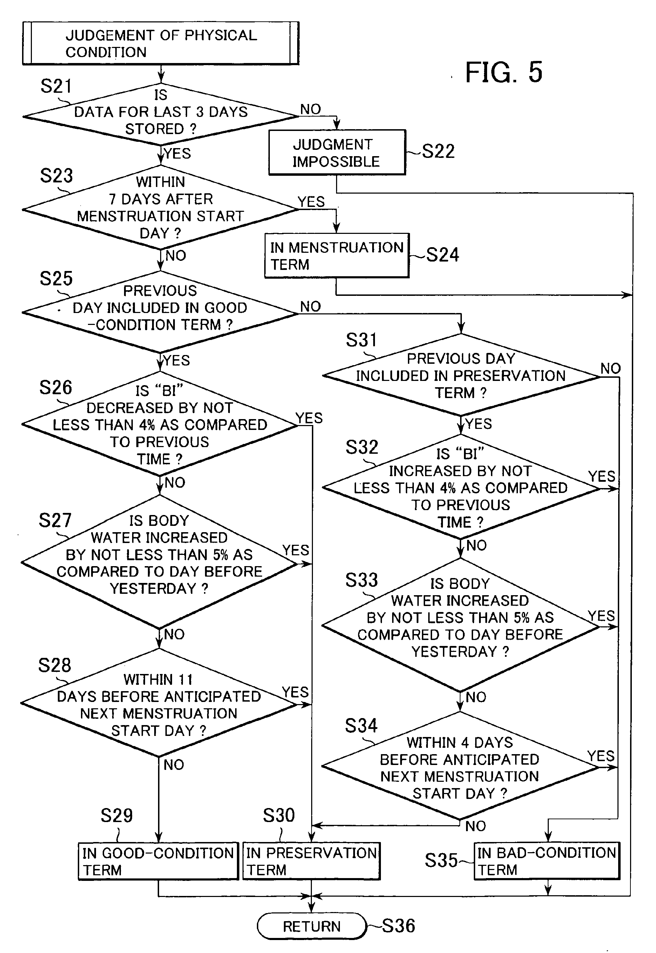

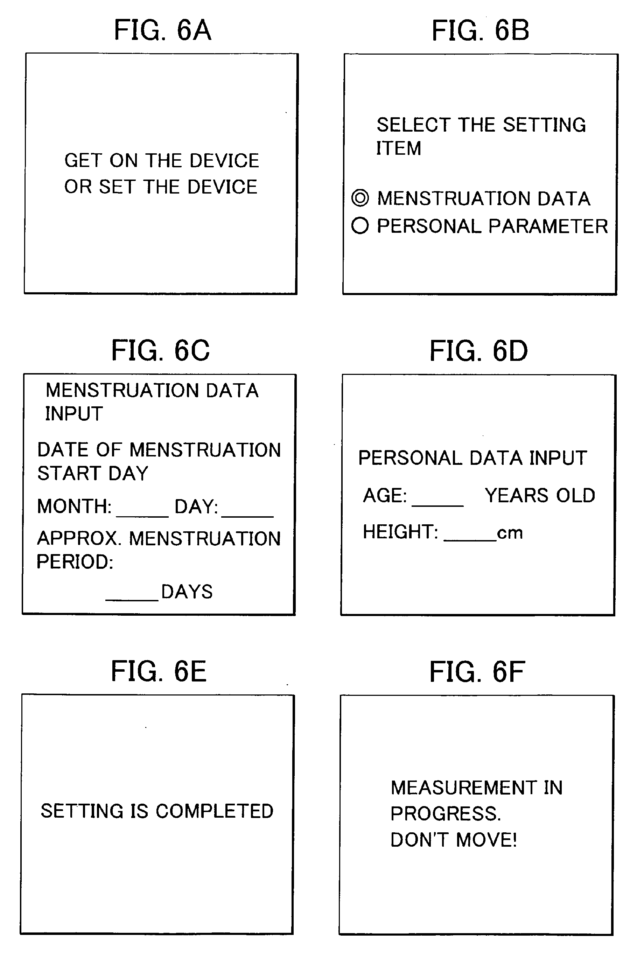

Figs. 4 and 5 are flow chart illustrating operation of the device 1 for prompting to control physical condition for a woman according to the present invention, and Figs. 6A to 8B each shows an example of content of display on the display unit 2.

First of all, the power switch 8 is depressed to turn the device 1 ON (step S1). Then, the device 1 is initialized so that the microcomputer 10 enters operation mode (step S2). Then, the display unit 7 displays the initial screen, as shown in Fig. 6A (step S3).

Next, the setting switch 6C is depressed to enter setting mode (step S4) so that the display unit 7 displays the screen for entering personal information, as shown in Fig. 6B (step S5).

On the screen for entering personal information, as shown in Fig. 6B, the user selects the item that she wants to enter the information. In particular, the item is selected using the up-switch 6A or the down-switch 6B. Then, by depressing the setting switch 6C, the item is determined. If it is determined to enter information about menstruation (step S6) then the display unit 7 displays the screen to prompt to enter information about menstruation, as shown in Fig. 6C (step S7). Then, the user enters the date of previous menstruation start day and the approx. number of days for the menstruation period that she knows by herself. The device 1 now considers the date of previous menstruation start day as the date of first day for the new and current menstruation period and calculates the anticipated date of next menstruation start day (step S8).

On the other hand, if it is determined to enter personal body information (step S9) then the display unit 7 displays the screen to prompt to enter personal parameters, as shown in Fig. 6 D (step S10).

When entering of the menstruation information and personal parameters is terminated then the display unit 7 displays a message informing that the setting mode is completed, as shown in Fig. 6E (step S11).

If the setting switch 6C is not depressed at step S4 the device 1 checks to determine whether the user gets on the device using the signal output from the load detector unit 28 (step S12). If a load not less than the predetermined value is detected then the device considers that the user gets on the device, and the display unit 7 displays a message, as shown in Fig. 6 F. The load signal output from the load detector unit 28 is amplified in the amplifier 29 and is sent to the A/D converter 31 via the switching unit 30. The A/D converter 31 converts the load signal from analog form into digital form, which is then sent to the microcomputer 10. The microcomputer 10 calculates body weight of the user from the input signal (step S13).

On the other hand, if no load is detected at step S12 the routine returns to step S3 wherein the display unit 7 displays the initial screen, as shown in Fig. 6A.

After completion of measurement of body weight, measurement of bioelectrical impedance is performed (step S 14). The microcomputer 10 supplies the signal to the filter circuit 21 according to measurement control parameter previously stored in ROM within the microcomputer 10. Then, the filter circuit 21 filters the signal to produce a living body applied signal which is sent to the AC current output circuit 22. The circuit 22 then converts the signal into a fixed RMS signal, which is then applied to the user via the current supplying electrodes 2A and 2B. As described above, the electrode 2B is connected to one output terminal of the AC current output circuit 22 via the reference resistor 32 and the electrode 2Ais connected to the other output terminal of the AC current output circuit 22. While the current is applied to the user an electric potential is detected across a pair of voltage measurement electrodes 3A and 3B which are contact to the user. The electric potential detected is sent to the differential amplifier 27 which produces an electric potential difference between both feet of the user. Furthermore, while the current is applied to the user the differential amplifier 26 produces an electric potential difference across the reference resistor 23. The electric potential difference signals from the differential amplifiers 26 and 27 are switched by the switching unit 30 under the control of the microcomputer 10 so that they are sent to the A/D converter 31. Then, the A/D converter 31 converts the analog signal sent thereto into digital signal which is then sent to the microcomputer 10. Thereafter, the microcomputer 10 calculates bioelectrical impedance on the basis of the digital signal sent thereto (step S14).

The calculated bioelectrical impedance is corrected based on the body weight measured in step S13 using the following correction equations to produce a body weight corrected bioelectrical impedance ("BI").

Correction Equation 1:

Body Weight Corrected "BI" = "BI" + "A" × Difference between Body Weight Measured First Time; and

Correction Equation 2:

Body Weight Corrected "BI" = "BI" + "B" × Difference between Body Weight Measured Last Time.

In other words, the body weight corrected "BI" is provided by addition or subtraction of increase/decrease in "BI" associated with increase/decrease in body weight to and from "BI" using either correction equation 1 or correction equation 2. Here, "A" and "B" are a correction coefficient. As the result, the body weight corrected "BI" from which any effect of change in body weight is removed is provided. It is noted that the term "bioelectrical impedance" used for judgment for physical condition of a woman that would be occurred at the period of a month, as described later, means the body weight corrected "BI".

Furthermore, measurement of bioelectrical impedance is performed using AC current at multiple frequencies. Now, measurement of bioelectrical impedance using AC current at multiple frequencies will be described in more detail.

The multi-frequency bioelectrical impedance measurement is repeated by "n" times that is preset, beginning with the frequency Fi = 1. In initial setting for measurement at first frequency the setting of "i = 1" is performed. The frequency "Fi" is set by the value of "i". On the basis of measurement control parameters already stored in ROM within the microcomputer 10 (hereafter referred to as "measurement control parameters"), the frequency of output signal is set, which is then sent to the AC current output circuit 22 via the filter circuit 21. The output current is applied to the person under test via the current supplying electrodes 2A and 2B.

Then, the current flowing through the person under test is detected by the reference resistor 23 separately provided of which analog output signal is converted into digital signal by the A/D converter 31, which digital signal is then stored in RAM of the microcomputer 10. At the same time, the voltage is picked up by the voltage measurement electrodes 3A and 3B that are contact with the person under test, and then, it is sent to a differential amplifier 27, which produces a signal showing difference between the voltage signals sent thereto, which signal is then sent to the A/D converter 31 via the switching unit 30. The A/D converter 31 converts analog input signal into digital signal, thereby measuring the bioelectrical impedance, which is then stored in RAM in the microcomputer 10.

After the measurement of bioelectrical impedance at first frequency has been done setting of "i = i + 1" is performed and a check is made to determine whether the measurement has been repeated by the predetermined number of times. If "i" is exceeded the predetermined number of times "n" then the measurement of bioelectrical impedance is terminated. But, if not exceeded, the measurement of bioelectrical impedance at the next frequency is performed.

Then, the impedance vector locus and the parameters associated therewith are calculated, based on the measurement values. Method of calculation of the impedance vector locus and the parameters associated therewith will briefly be described, here.

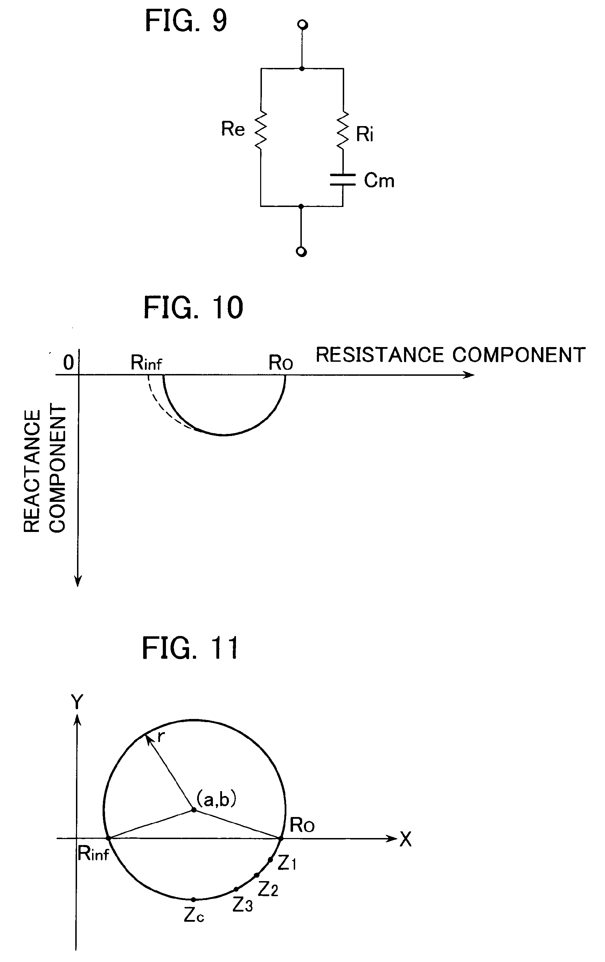

The bioelectrical impedance is normally represented by an equivalent circuit having concentrated constants consisting of extra-cellular water resistance "Re", intra-cellular water resistance "Ri" and cell membrane capacity "Cm", as shown in Fig. 9. Actually, the individual cells forming a living body are represented by the circuits each having different constant depending on the difference in geometry or characteristic thereof. Therefore, the living body made up of collection of those cells does not produce an impedance vector locus of half-circle as in the case of measurement of the equivalent circuit having concentrated constants, but produces a locus of an arc according to "Cole-Cole's model". As the result, the bioelectrical impedance generally provides the locus of arc, as shown in Fig. 10. From the assumption that the vector locus of arc is desired the bioelectrical impedance measurement values Z1, Z2,.....Zn at the frequencies "Fi" (i = 1 ～ n) are obtained, as plotted in Fig. 11. In this case, the real axis (or the horizontal axis) of the impedance plane is defined as "X" axis and the imaginary axis (or the vertical axis) is defined as "Y" axis.

From "Zi" (i = 1 ～ n) as plotted on the coordinate, the following correlation function is derived.

$$(X - a)^2 + (Y - b)^2 = r^2$$

where "a" is "X" coordinate at the center of a circle; "b" is "Y" coordinate at the center of the circle; and "r" is radius of the circle. Thus, the equation is an approximated correlation function among "n" points. Accordingly, the following equation is derived.

$$X = a \pm \sqrt{(r^2 - b^2)}$$

Furthermore, because of Ro > Rinf, they are written as follows:

$$Ro = a + \sqrt{(r^2 - b^2)}$$

$$Rinf = a - \sqrt{(r^2 - b^2)}$$

Therefore, "Re" and "Ri" in the equivalent circuit in Fig. 9 are written as follows:

$$Re = Ro$$

$$Ri = Ro \cdot Rinf/(Ro - Rinf)$$

The impedance vector "Zc" at the characteristic frequency "Fc" is at such point that the absolute value of reactance, i.e., imaginary axis component or "Y" axis component reaches the maximum. Accordingly, "X" coordinate of the real axis component and "Y" coordinate of the imaginary axis component are written as follows:

$$X = a, Y = b - r$$

In other words, it may be written as follows:

$$Zc = a + j (b - r)$$

Thereby, the resistance component "R" and the reactance component "X" can be derived.

Depending on the derived impedance vector locus and the associated parameters such as "Ro" and "Rinf' or "Re" and "Ri" as well as the entered personal parameters such as sex, height, body weight, age, the intra-cellular water "ICW", the extra-cellular water "ECW", the ration of intra-cellular/extra-cellular water "ICW/ECW", the total body water "TBW" (=ICW + ECW), etc. can be derived according to the well known calculation method. For example, the intra-cellular water "ICW", the extra-cellular water "ECW", and the total body water "TBW" are derived by the following equations using "Ri", "Re", height "Ht" and body weight "W".

$$ICW = K_{i1} \ Ht^2 / Ri + K_{i2} \ W + K_{i3}$$

$$ECW = K_{e1} \ Ht^2 / Re + K_{e2} \ W + K_{e3}$$

$$TBW = ICW + ECW$$

(where $K_{i1}$, $K_{i2}$, $K_{i3}$, $K_{e1}$, $K_{e2}$, $K_{e3}$ are a coefficient.)

On the basis of those measurement and calculations the total body water can be calculated (step S15). If it is observed that the calculated total body water is increased by not less than 5% as compared to average of total body water for the current menstruation period then it is determined that a swell has been occurred at the time of measurement.

Next, judgment for physical condition of a woman under test is conducted.

In the device 1 for prompting to control physical condition for a woman according to the present invention judgment for physical condition of a woman that would be occurred at the period of a month is performed in order to determine to which term she now belongs according to the following definition for the terms:

(1) Date of menstruation start day that is entered is defined as the menstruation start day;
(2) Interval of one week after the menstruation start day is defined as the first term (i.e. menstruation term);
(3) Interval of at least five days after the first term is defined as the second term (i.e. good-condition term);
(4) Interval up to one day before the day at which "BI" of 4% lower than the average "BI" in the second term (i.e. good-condition term) of the current period is measured or at which total body water of 5% higher than the average total body water for five days after completion of the first term is calculated is defined as the second term (i.e. good-condition term), and the last day thereof is defined as the ovulation day;

(5) Interval after the second term up to one week before the anticipated next menstruation start day estimated from the previous data (i.e. average number of days for previous menstruation periods) is defined as the third term (i.e. preservation term);

(6) Interval after the third term up to the date of next menstruation start day that is entered is defined as the fourth term (i.e. bad-condition term);

(7) Alternatively, the fourth term (i.e. bad-condition term) may be defined to start at such day in the third term (i.e. preservation term) that total body water is increased by not less than 5% as compared to the previous day.

More concretely, the device 1 conducts judgment for physical condition according to a physical condition judgment routine as follows (step S17):

At first, it is checked to determine whether the data measured within last three days has been stored in the auxiliary storage unit 14 (step S21). The reason for which is that the present device is designed to conduct measurement every day for judgment for physical condition based on the change in bioelectrical impedance and total body water, but, even if measurement could not be conducted on the previous day the data within last three days, if stored in the storage unit, can be used for judgment. However, if no data within last three days has been stored in the storage unit it is determined that judgment can't be performed at all (step S22).

If the data within last three days has been stored in the storage unit at step S21 then a first phase for judgment is conducted to determine whether the woman under test is positioned within the interval of 7 days after the menstruation start day (step S23). If so, the woman under test is determined to belong to the menstruation term (step S24).

If the woman under test is not positioned within the interval of 7 days after the menstruation start day at step S23 then it is checked to determine whether physical condition of the woman under test on the previous day (or on the previous measurement day) belongs to good-condition term (step S25). If so, it is checked to determine whether the good-condition term still continues. Then, it is checked to determine whether the currently measured "BI" (at measurement frequency of 50 kHz) is decreased by not less than 4% as compared to the average "BI" in the good-condition term of the current period that has been stored in RAM (step S26).

Furthermore, it is checked to determine whether the currently measured total body water is increased by not less than 5% as compared to the average total body water in the current period (step S27). This increase in body water corresponds to such portion of a curve illustrating the body water in Fig. 1 that is indicated by a broken line.

Then, it is checked to determine whether the woman under test is now positioned within the interval of eleven (11) days before the anticipated date of next menstruation start day, which is resulted from calculation of number of days for the menstruation period (step S28). This is for the purpose of checking the anticipated date of ovulation day resulting from simple calculation of number of days, because there is possibility of insufficient check for ovulation simply by measurement of "BI" and body water mass.

If all the answers in steps S26 to S28 are "NO" the woman under test is considered to still belong to good-condition term (step S29).

If any one of the answers in steps S26 to S28 is "YES" the woman under test is considered that ovulation is occurred to shift to the preservation term (step S30).

If the woman under test does not belong to the good-condition term at step S25 then it is checked to determine whether physical condition of the woman under test on the previous day belongs to the preservation term (step S31). If so, it is checked to determine whether "BI" is increased by not less than 4% as compared to the average "BI" in the preservation term of the current period (step S32). This is for the purpose of checking whether "BI" is increased due to occurrence of swell.

Then, it is checked to determine whether total body water is increased by not less than 5% as compared to that on the day before yesterday (step S33). This is also for the purpose of checking whether any swell has occurred depending on increase in total body water.

Then, it is checked to determine whether the woman under test is now positioned within the interval of four (4) days before the anticipated date of next menstruation start day, which is resulted from calculation of number of days for the menstruation period (step S34). This is for the purpose of checking the terms before menstruation, resulting from simple calculation of number of days, because there may be some women who are not necessarily to have significant swell (accumulation of body water) before menstruation, in which case it is difficult to judge the terms before menstruation simply by change in "BI" and total body water.

If all the answers in steps S32 to S34 are "NO" the woman under test is considered to still belong to the preservation term (step S30).

If any one of the answers in steps S32 to S34 is "YES" the woman under test is considered to belong to the bad-condition term before menstruation (step S35). If the woman under test does not belong to the preservation term at step S31 the routine proceeds to step S35 due to the bad-condition term still continued.

As described above, the current physical condition of the woman under test is judged according to the physical condition judgment routine (step S36).

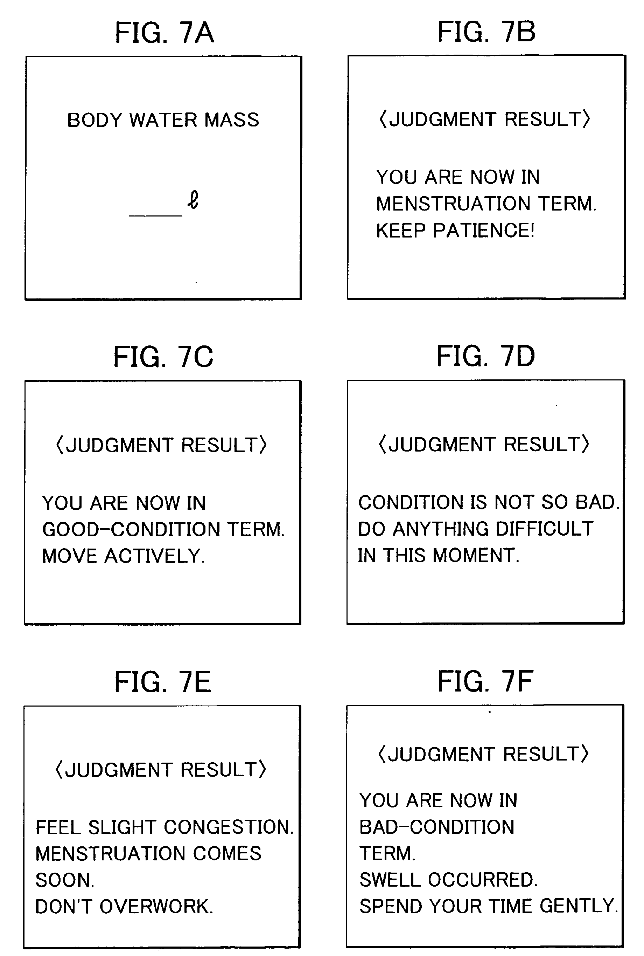

Referring again to Fig. 4, the result of judgment for physical condition of the woman under test is displayed on the display unit 12. Figs 7B to 7F show the results of judgment. In particular, Fig. 7B is a screen illustrating the information for menstruation term; Fig. 7C illustrates the information for good-condition term; Fig. 7D illustrates the information for preservation term; Fig. 7E illustrates the information for bad-condition term; and Fig. 7F illustrates occurrence of swell in the bad-condition term.

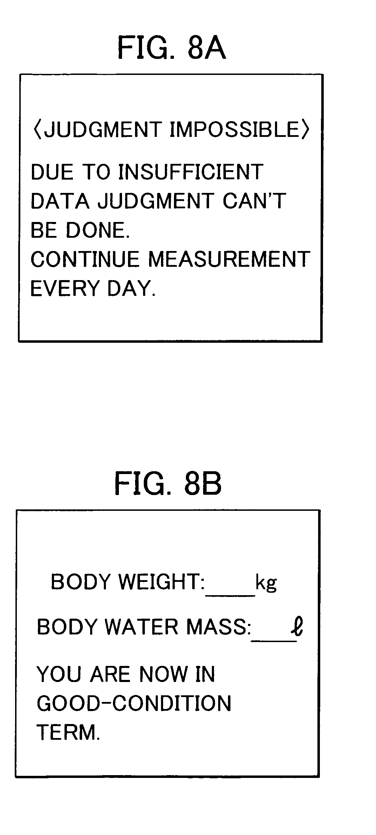

In case where judgment for physical condition can't performed then a message informing such fact is displayed, as shown in Fig. 8A.

At a fixed time period elapsed after displaying the judgment result the currently measured body weight, total body water and physical condition of the woman under test are displayed. Fig. 8B illustrates the information for good-condition term of the woman under test, for example.

After displaying the judgment result the currently measured data is transferred from RAM in the microcomputer 10 to the auxiliary storage unit 14 and is stored therein together with the date data (step S19). Thereafter, when a fixed time period is elapsed the information on the display unit 12 disappears and the present device 1 is turned OFF (step S20).

The embodiments of the present invention have been described above. However, the numerical values used for definition of the terms in the above mentioned embodiments are simply for the purpose of illustration and without any limitation. It is possible to modify those numerical values according to the precision with which the judgment is made, without departing from the scope of the present invention.

In the above embodiments the present device has been described as having capability of judging physical condition of a woman that would be occurred at the period of a month, based on the transition in bioelectrical impedance and total body water. However, the present invention is not limited to such embodiments. For example, the present device may be embodied to conduct judgment for physical condition, based on only the transition in total body water, or based on any combination of calculation of number of days and the transition in total body water. In such case, if it is observed that total body water is increased at several days before the anticipated date of the next menstruation start day then it can be judged that the woman under test becomes belonging to the bad-condition term.

In the above configuration data of menstruation start day has been entered, but it is not necessary. Alternatively, menstruation term, ovulation day and other terms may be determined only depending on the transition in total body water and bioelectrical impedance.

Moreover, in the above embodiments, the present device has been described as having capability of calculating total body water depending on measurement of bioelectrical impedance at multiple frequencies. Alternatively, total body water may be calculated depending on measurement of bioelectrical impedance at single frequency. In such case, by correction of the bioelectrical impedance with body weight, and by calculation of total body water using the corrected bioelectrical impedance, any change in impedance due to any change in body weight can be compensated for to provide higher precision with which total body water is calculated.

In addition, the present device has been described as having function of measuring body weight of the woman under test. Alternatively, a bioelectrical impedance measurement unit may be provided separately with a body weight measurement unit so that measurement data is sent by wireless communication for judgment and control. Or otherwise, body weight data may manually be entered via key switches.

Furthermore, both feet of the woman under test have been described as the parts between which measurement of bioelectrical impedance is conducted in the above mentioned embodiments. However, the present invention is not limited to such parts. For example, other parts such as between both hands and between a hand and a foot may be selected for measurement of bioelectrical impedance.

Additionally, the present device may have an additional function of calculation and display of indices such as body fat rate, body fat mass, visceral fat mass, muscle mass, basal metabolism, bone mass, etc. which can be calculated from measurement of body weight and bioelectrical impedance.

Furthermore, the present device comprises an external input / output interface, as has been described in the above embodiment. Therefore, it may be communicated with some external terminals via such interface. For example, measurement result from the present device may be sent to a portable phone, PDA or other information terminal, which are then accessible to some web sites. In such case more useful information can be provided. In particular, information such as meal, beauty, exercise, etc. may be provided in association with the judged terms within a menstruation period so that the user can receive any suitable advice according to her physical condition.

**Claims**

1. A device for prompting to control physical condition for a woman, comprising:

   an impedance measurement unit;
   a body weight input unit;
   a total body water calculation unit;
   a total body water storage unit; and
   a physical condition judgment unit, wherein

said impedance measurement unit measures bioelectrical impedance of a woman under test,

said body weight input unit enters body weight of the woman under test,

said total body water calculation unit calculates total body water of the woman under test, based on the measured bioelectrical impedance and the entered body weight,

said total body water storage unit stores the calculated total body water of the woman under test, and

said physical condition judgment unit judges any change in physical condition of the woman that would be occurred at the period of a month, based on the transition in total body water stored in said storage unit.

2. A device for prompting to control physical condition for a woman according to claim 1 in which it further comprises a bioelectrical impedance correction unit, wherein

said bioelectrical impedance correction unit corrects the measured bioelectrical impedance with the body weight entered by said body weight input unit, and

said total body water calculation unit calculates total body water of the woman under test, based on the corrected bioelectrical impedance and the entered body weight.

3. A device for prompting to control physical condition for a woman according to claim 1 in which it further comprises a bioelectrical impedance storage unit, wherein

said bioelectrical impedance storage unit stores the measured bioelectrical impedance, and

said physical condition judgment unit judges any change in physical condition of the woman that would be occurred at the period of a month, based on the transition in total body water and bioelectrical impedance stored in said storage unit.

4. A device for prompting to control physical condition for a woman according to claim 3 in which it further comprises a bioelectrical impedance correction unit, wherein

said bioelectrical impedance correction unit corrects the measured bioelectrical impedance with the body weight entered by said body weight input unit,

said bioelectrical impedance storage unit stores the corrected bioelectrical impedance, and

said physical condition judgment unit judges any change in physical condition of the woman that would be occurred at the period of a month, based on the transition in total body water and corrected bioelectrical impedance stored in said storage unit.

5. A device for prompting to control physical condition for a woman according to any one of claims 1 to 4 in which said body weight input unit is a body weight measurement unit so that the measured body weight is used for the entered body weight.

6. A device for prompting to control physical condition for a woman according to any one of claims 1 to 4 in which said physical condition judgment unit judges an occurrence of swell by comparison between the measured total body water and the stored total body water.

7. A device for prompting to control physical condition for a woman

according to any one of claims 1 to 4 in which it further comprises a display unit, wherein

said display unit displays the judged physical condition together with the current total body water.

8. A device for prompting to control physical condition for a woman according to any one of claims 1 to 4 in which it further comprises an input unit, wherein

said input unit enters data of menstruation start day, and

said physical condition judgment unit judges any change in physical condition of the woman that would be occurred at the period of a month, based on the entered data of menstruation start day.

9. A device for prompting to control physical condition for a woman according to any one of claims 1 to 4 in which said physical condition judgment unit judges any change in physical condition of the woman that would be occurred at the period of a month, by comparison of the current total body water with the average of previously measured total body water.

10. A device for prompting to control physical condition for a woman according to claim 3 or 4 in which said physical condition judgment unit judges any change in physical condition of the woman that would be occurred at the period of a month, by comparison of the current total body water with the average of previously measured total body water, and by comparison of current bioelectrical impedance with the average of previously measured bioelectrical impedance.

11. A device for prompting to control physical condition for a woman according to claim 9 in which the average of total body water used by said physical condition judgment unit is an average of total body water after termination of the current menstruation, and if there is an increase of not less than the predefined value, as indicated by comparison of the current total body water with that average of the total body water, it is determined that ovulation has been occurred.

**12.** A device for prompting to control physical condition for a woman according to claim 9 in which the average of total body water used by said physical condition judgment unit is an average of total body water after ovulation, and if there is an increase of not less than the predefined value, as indicated by comparison of the current total body water with that average of the total body water, it is determined that physical condition is the one that is occurred immediately before the occurrence of next menstruation.

**13.** A device for prompting to control physical condition for a woman according to any one of claims 1 to 4 in which said physical condition judgment unit anticipates the next ovulation day and the next menstruation start day using previous data of number of days for menstruation period.

# FIG. 1

| MENSTRUATION START | MENSTRUATION END | OVULATION | | PMS | MENSTRUATION START |

MENSTRUATION TERM · GOOD-CONDITION TERM · PRESERVATION TERM · BAD-CONDITION TERM

B1

TOTAL BODY WATER

0    7    14    21    28

(NUMBER OF DAYS)

EP 1 504 717 A1

# FIG. 2

# FIG. 3

FIG. 4

```
S1─(POWER ON)

S2─[INITIALIZATION]

S3─[INITIAL SCREEN]                    S12  ◇LOAD
                                            DETECTION?─NO
S4  ◇SETTING?─NO
         │YES                               │YES
                                       S13─[MEASURE BODY
S5─[PERSONAL                               WEIGHT]
    INFORMATION
    INPUT SCREEN]                     S14─[MEASURE
                                           BIOELECTRICAL
                                           IMPEDANCE]
S6  ◇MENSTRUATION
     INPUT?─NO                         S15─[CALCULATE BODY
         │YES                               WATER MASS]
S7─[MENSTRUATION
    INPUT SCREEN]                      S16─[RETRIEVE
                                           BIOELECTRICAL
S8─[CALCULATE                               IMPEDANCE & BODY
    NUMBER OF                                WATER MASS UP TO
    DAYS FOR                                 PREVIOUS DAY]
    MENSTRUATION]
S11─[SETTING                           S17─[JUDGE PHYSICAL
     COMPLETION                              CONDITION]
     SCREEN]
                                       S18─[DISPLAY RESULT]
S9  ◇PERSONAL
     PARAMETER                         S19─[STORE DATA]
     INPUT?─NO
         │YES  S10
S10─[HEIGHT & AGE                      S20─(POWER OFF)
     INPUT]
```

16

## FIG. 5

```
        ┌─────────────────────────────┐
        │ JUDGEMENT OF PHYSICAL       │
        │ CONDITION                   │
        └─────────────────────────────┘
                      │
   S21          ╱IS           ╲        NO
        ╱ DATA FOR LAST 3 DAYS ╲──────────┐
        ╲     STORED ?         ╱          ▼
                │ YES              ┌──────────────┐
                │                  │ JUDGMENT     │ S22
   S23      ╱  WITHIN  ╲          │ IMPOSSIBLE   │
        ╱  7 DAYS AFTER ╲    YES   └──────────────┘
        ╲ MENSTRUATION START ╲────────┐
        ╲     DAY ?         ╱         ▼
                │ NO            ┌──────────────┐
                │               │ IN MENSTRUATION │ S24
   S25      ╱ PREVIOUS ╲        │ TERM         │
        ╱ DAY INCLUDED IN GOOD╲  NO  └──────────────┘
        ╲ -CONDITION TERM ?  ╱─────────┐
                │ YES                   ▼
                                 S31  ╱ PREVIOUS DAY ╲   NO
   S26     ╱ IS "BI" ╲          ╱ INCLUDED IN PRESERVATION ╲──┐
        ╱ DECREASED BY NOT ╲    ╲    TERM ?    ╱              │
        ╲ LESS THAN 4% AS COMPARED╲ YES  │ YES                │
        ╲ TO PREVIOUS ╱──────┐     S32  ╱ IS "BI" ╲          │
        ╲   TIME ?  ╱        │     ╱ INCREASED BY NOT ╲  YES  │
                │ NO         │     ╲ LESS THAN 4% AS COMPARED ╲─┤
   S27     ╱ IS BODY ╲       │     ╲ TO PREVIOUS ╱            │
        ╱ WATER INCREASED ╲  │     ╲   TIME ?  ╱              │
        ╲ BY NOT LESS THAN 5% AS╲ YES │ NO                   │
        ╲ COMPARED TO DAY BEFORE╲──┤  S33  ╱ IS BODY ╲       │
        ╲  YESTERDAY ? ╱        │  ╱ WATER INCREASED ╲  YES  │
                │ NO            │  ╲ BY NOT LESS THAN 5% AS ╲─┤
   S28     ╱ WITHIN 11 ╲        │  ╲ COMPARED TO DAY BEFORE╲ │
        ╱ DAYS BEFORE ANTICIPATED╲ │  ╲ YESTERDAY ? ╱       │
        ╲ NEXT MENSTRUATION ╲  YES │      │ NO              │
        ╲ START DAY ? ╱─────────┤  S34  ╱ WITHIN 4 DAYS ╲   │
                │ NO            │  ╱ BEFORE ANTICIPATED ╲ YES│
                                │  ╲ NEXT MENSTRUATION ╲────┤
   S29         S30              │  ╲ START DAY ? ╱          │
┌──────────────┐┌──────────────┐│      │ NO               │
│ IN GOOD-CONDITION││IN PRESERVATION│   S35  ┌──────────────┐
│ TERM         ││ TERM         │       │ IN BAD-CONDITION│
└──────────────┘└──────────────┘       │ TERM         │
        │         │                    └──────────────┘
        └─────────┴──────────┬──────────────┘
                      ┌──────────────┐
                      │   RETURN     │ S36
                      └──────────────┘
```

## FIG. 6A

GET ON THE DEVICE
OR SET THE DEVICE

## FIG. 6B

SELECT THE SETTING
ITEM

◎ MENSTRUATION DATA
○ PERSONAL PARAMETER

## FIG. 6C

MENSTRUATION DATA
INPUT

DATE OF MENSTRUATION
START DAY

MONTH: _____ DAY: _____

APPROX. MENSTRUATION
PERIOD:

_____ DAYS

## FIG. 6D

PERSONAL DATA INPUT

AGE: _____ YEARS OLD

HEIGHT: _____ cm

## FIG. 6E

SETTING IS COMPLETED

## FIG. 6F

MEASUREMENT IN
PROGRESS.
DON'T MOVE!

# FIG. 7A

BODY WATER MASS

____ ℓ

# FIG. 7B

〈JUDGMENT RESULT〉

YOU ARE NOW IN
MENSTRUATION TERM.
KEEP PATIENCE!

# FIG. 7C

〈JUDGMENT RESULT〉

YOU ARE NOW IN
GOOD-CONDITION TERM.
MOVE ACTIVELY.

# FIG. 7D

〈JUDGMENT RESULT〉

CONDITION IS NOT SO BAD.
DO ANYTHING DIFFICULT
IN THIS MOMENT.

# FIG. 7E

〈JUDGMENT RESULT〉

FEEL SLIGHT CONGESTION.
MENSTRUATION COMES
SOON.
DON'T OVERWORK.

# FIG. 7F

〈JUDGMENT RESULT〉

YOU ARE NOW IN
BAD-CONDITION
TERM.
SWELL OCCURRED.
SPEND YOUR TIME GENTLY.

# FIG. 8A

⟨JUDGMENT IMPOSSIBLE⟩

DUE TO INSUFFICIENT
DATA JUDGMENT CAN'T
BE DONE.
CONTINUE MEASUREMENT
EVERY DAY.

# FIG. 8B

BODY WEIGHT:____kg

BODY WATER MASS:____$\ell$

YOU ARE NOW IN
GOOD-CONDITION
TERM.

## FIG. 9

## FIG. 10

## FIG. 11

European Patent
Office

**EUROPEAN SEARCH REPORT**

Application Number

EP 04 01 7890

| | DOCUMENTS CONSIDERED TO BE RELEVANT | | |
|---|---|---|---|
| Category | Citation of document with indication, where appropriate, of relevant passages | Relevant to claim | CLASSIFICATION OF THE APPLICATION (Int.Cl.7) |
| X<br><br>Y | US 6 587 716 B2 (KODAMA MIYUKI)<br>1 July 2003 (2003-07-01)<br>* column 4, line 14 - line 20 *<br><br>* column 5, line 11 - line 15 *<br>* column 6, line 1 - line 34 *<br>* column 7, line 14 - line 53 *<br>* column 8, line 14 - line 49 *<br>* column 9, line 59 - column 10, line 9 *<br>----- | 1,3,5,7,<br>9<br><br>2,4,6,8,<br>13 | A61B5/053<br>A61B10/00 |
| Y | US 6 402 699 B1 (KODAMA MIYUKI ET AL)<br>11 June 2002 (2002-06-11)<br>* column 1, line 14 - line 18 *<br>* column 5, line 1 - line 55 *<br>* column 6, line 19 - line 46 *<br>----- | 2,4,6,8 | |
| Y | US 4 685 471 A (FERNANDO RANJIT S ET AL)<br>11 August 1987 (1987-08-11)<br>* column 1, line 27 - line 30 *<br>----- | 13 | |
| | | | TECHNICAL FIELDS SEARCHED (Int.Cl.7)<br><br>A61B |

The present search report has been drawn up for all claims

| Place of search | Date of completion of the search | Examiner |
|---|---|---|
| Munich | 3 December 2004 | Martelli, L |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons
............................................................
& : member of the same patent family, corresponding
document

EPO FORM 1503 03.82 (P04C01)

**ANNEX TO THE EUROPEAN SEARCH REPORT
ON EUROPEAN PATENT APPLICATION NO.**   EP 04 01 7890

This annex lists the patent family members relating to the patent documents cited in the above-mentioned European search report.
The members are as contained in the European Patent Office EDP file on
The European Patent Office is in no way liable for these particulars which are merely given for the purpose of information.

03-12-2004

| Patent document cited in search report | | Publication date | Patent family member(s) | | Publication date |
|---|---|---|---|---|---|
| US 6587716 | B2 | 16-05-2002 | JP | 2002177235 A | 25-06-2002 |
| | | | EP | 1213585 A2 | 12-06-2002 |
| | | | US | 2002058880 A1 | 16-05-2002 |
| US 6402699 | B1 | 11-06-2002 | JP | 2001078977 A | 27-03-2001 |
| | | | CA | 2318652 A1 | 13-03-2001 |
| | | | CN | 1292248 A | 25-04-2001 |
| | | | EP | 1084676 A1 | 21-03-2001 |
| | | | ID | 27242 A | 15-03-2001 |
| | | | TW | 568767 B | 01-01-2004 |
| US 4685471 | A | 11-08-1987 | AT | 49701 T | 15-02-1990 |
| | | | DE | 3575517 D1 | 01-03-1990 |
| | | | EP | 0177994 A2 | 16-04-1986 |
| | | | US | 4770186 A | 13-09-1988 |
| | | | JP | 61113437 A | 31-05-1986 |
| | | | US | 4836216 A | 06-06-1989 |